# EUROPÄISCHE PATENTSCHRIFT

(11) **EP 0 689 821 B1**
(45) Veröffentlichungstag und Bekanntmachung des Hinweises auf die Patenterteilung: **09.02.2000**
(21) Anmeldenummer: 95109135.4
(22) Anmeldetag: 13.06.1995
(51) Int. Cl.: A61F 13/56

(54) **Saugfähige Einlage**
Absorbent catamenial device
Dispositif cataménial absorbant

(30) Priorität: 14.06.1994 JP 13197394
(43) Veröffentlichungstag der Anmeldung: 03.01.1996
(73) Patentinhaber: UNI-CHARM CORPORATION, Kawanoe-shi Ehime-ken (JP)
(72) Erfinder: Mizutani, Satoshi, Kawanoe-shi, Ehime-ken (JP)
(74) Vertreter: Sperling, Rüdiger, Dipl.-Ing.

(56) Entgegenhaltungen:
- EP-A- 0 025 611
- WO-A-92/04000
- GB-A- 2 119 656

## Beschreibung

Die vorliegende Erfindung betrifft eine saugfähige Einlage und insbesondere eine saugfähige Einlage, die als eine Menstruationseinlage, eine Inkontinenzeinlage und ähnliches geeignet ist.

Herkömmliche saugfähige Einlagen, wie etwa Menstruationseinlagen bzw. Binden, haben eine Befestigungseinrichtung, die auf ihrer Rückseite angeordnet ist, so daß die Einlage an dem Höschen durch die Befestigungseinrichtung befestigt werden kann und dadurch auch während aktiver Körperbewegungen der Trägerin an ihrer Zielposition gehalten werden. Beispielsweise zeigt die japanische offengelegte Gebrauchsmusteranmeldung Nr. 1984-177425 eine Technik auf, gemäß welcher eine Abmessung in Querrichtung einer Befestigungseinrichtung in Längsrichtung von wenigstens einem Ende zum Mittelbereich einer Menstruationseinlage hin allmählich verringert wird. Eine derartige Befestigungseinrichtung wirkt in derselben Weise wie die Befestigungszonen.

Wenn eine relative Position einer Menstruationseinlage und des Höschens der Trägerin sich bedingt durch die Bewegung der Trägerin verschiebt, kann die Einlage verdreht oder gefaltet werden, was nicht nur ein unkomfortables Tragegefühl verursacht, sondern auch das teilweise Herausragen der Einlage aus dem Höschen, wobei die Bekleidung mit Menstruationsflüssigkeit verschmiert werden kann. Ein derartiges Problem tritt wahrscheinlich im Schrittbereich des Höschens auf. Die in der vorstehend genannten Anmeldung aufgezeigte Einlage schlägt sicherlich die Befestigungseinrichtung vor, lehrt jedoch keine effektive Lösung dieses Problems. Gemäß dieser bekannten Technik wird eine Haltekraft der Befestigungseinrichtung so eingestellt, daß sie das Trennen bzw. das Abziehen der benutzten Einlage, die mit Menstruationsflüssigkeit verschmutzt ist, von dem Höschen erlaubt. Es ist jedoch der Fall, daß das Höschen oftmals nicht in engem Kontakt mit dem Körper der Trägerin ist, insbesondere in einem Bereich, der vom Schritt zur Hüfte verläuft, und zusätzlich die Hüfte häufig andere Objekte, wie etwa einen Stuhl streift, auf welchem die Trägerin sitzt. Folglich ist es oftmals schwierig, daß die Einlage zwischen dem Höschen und dem Körperbereich der Trägerin gehalten wird. Insbesondere eine aktive Bewegung des Körpers der Trägerin kann die Einlage relativ zu dem Höschen der Trägerin verschieben und die verschobene Einlage gibt der Trägerin ein unkomfortables Gefühl, insbesondere im Hüftbereich, und verschmutzt den Körper der Trägerin sowie die Kleidung mit Menstruationsflüssigkeit. Auch im Hinblick auf dieses Problem lehrt die vorstehend genannte Anmeldung keine wirksame Lösung.

Aus WO-A-92/04000 ist ebenfalls eine saugfähige Einlage beschrieben, die zur Fixierung Klebestreifen aufweist, wobei die Klebestreifen in Querrichtung nebeneinanderliegend angeordnet sind und von dem vorderen Abschnitt über den mittleren Abschnitt zum hinteren Abschnitt der Einlage verlaufen. Der in Querrichtung gemessene Abstand der nebeneinanderliegenden Klebestreifen ist hierbei in dem mittleren Abschnitt der Einlage geringer als in dem vorderen oder hinteren Abschnitt. Auch bei dieser bekannten Einlage ist die Haltekraft der Klebestreifen so bemessen, daß ein Abziehen der benutzten Einlage vom Höschen möglich ist. Nachteilig ist hieran, daß sich die Einlage bei körperlicher Aktivität der Trägerin leicht verschieben kann.

Der Erfindung liegt also die Aufgabe zugrunde, eine Einlage der vorstehend beschriebenen Art zu schaffen, die sich einerseits sicher fixieren läßt und andererseits von der Trägerin leicht entfernt werden kann.

Die Lösung der Aufgabe ergibt sich aus Patentanspruch 1. Unteransprüche zeigen bevorzugte Ausführungsformen der Erfindung.

Erfindungsgemäß wird die vorstehend dargelegte Aufgabe durch eine saugfähige Einlage gelöst, umfassend eine flüssigkeitsdurchlässige Decklage, eine flüssigkeitsundurchlässige Außenlage, einen zwischen diesen beiden Lagen angeordneten flüssigkeitssaugfähigen Kern und eine Befestigungseinrichtung, die an der Außenlage zur Befestigung der Einlage am Höschen vorgesehen ist, wobei die Befestigungseinrichtung Klebezonen umfaßt, die entlang in Querrichtung einander gegenüberliegenden Seiten der Einlage vorgesehen sind, wobei eine zwischen äußeren Rändern der in Querrichtung einander gegenüberliegenden Klebezonen gemessene Abmessung in einem in Längsrichtung vorderen und hinteren Abschnitt der Einlage größer ist als in einem in Längsrichtung mittleren Abschnitt der Einlage und eine zum Abziehen der Einlage erforderliche Kraft in dem hinteren Abschnitt höher ist als in den übrigen Abschnitten.

Die Einlage mit diesem Aufbau kann zuverlässig durch die Befestigungseinrichtung am Höschen nicht nur um den Schrittbereich, sondern auch entlang den Seitenrändern von Beinöffnungen in Längsrichtung im vorderen und hinteren Bereich des Schrittbereiches befestigt werden. Auf diese Weise wird der in Längsrichtung hintere Abschnitt der Einlage in ausreichender Weise gegen die Hüfte des Höschens stabilisiert, um sicherzustellen, daß auch eine aktive Bewegung des Körpers der Trägerin keine Verschiebung der Einlage verursacht und daß die Einlage, die durch Menstruationsflüssigkeit verschmutzt wurde, von den Höschen leicht abgezogen werden kann, beginnend von dem vorderen Abschnitt, in welchem die Klebekraft relativ schwach ist.
Fig. 1 ist eine Draufsicht, die eine Ausführungsform einer saugfähigen Einlage als Menstruationseinlage gemäß der Erfindung zeigt; und
Fig. 2 ist eine Ansicht ähnlich Fig. 1, die jedoch eine andere Ausführungsform der erfindungsgemäßen Einlage zeigt.

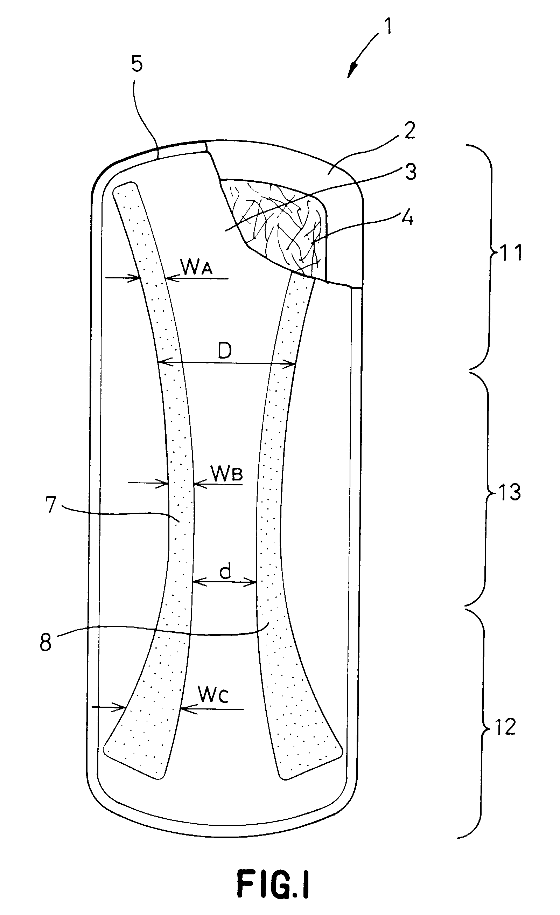

Wie Fig. 1 zeigt, umfaßt eine Menstruationseinlage 1 eine flüssigkeitsdurchlässige Decklage 2, eine flüssigkeitsundurchlässige Außenlage 3 und einen flüssigkeitssaugfähigen Kern 4, der zwischen diesen beiden Lagen 2, 3 angeordnet ist, wobei Abschnitte der Deck- und der Außenlage 2, 3, die sich über einen Umfangsrand des Kernes 4 hinaus nach außen erstrecken, miteinander entlang einer Verbindungslinie 5 wasserdicht verbunden sind. Die Rückseite der Einlage 1, die zum Anlegen an das getragene Höschen bestimmt ist, ist mit zwei Klebezonen versehen, die eine Befestigungseinrichtung bilden, die mit Heißschmelzkleber an der Außenlage 3 in Form von Streifen, Bändern oder Bahnen aufgetragen ist.

Es sei angenommen, daß die Einlage 1 in Längsrichtung in einen vorderen Abschnitt 11, einen Mittelabschnitt 13 und einen hinteren Abschnitt 12 eingeteilt ist. Klebezonen 7, 8 erstrecken sich in Längsrichtung vom vorderen Abschnitt 11 zum hinteren Abschnitt 12, wobei sie kreisförmige Bögen beschreiben, die bezüglich einer Mittellinie (nicht dargestellt), die die Einlage 1 in Querrichtung in eine rechte und eine linke Hälfte unterteilt, konvex sind. Eine Abmessung "D", gemessen zwischen den äußeren Rändern der jeweiligen Klebezonen 7, 8, ist in dem vorderen und dem hinteren Abschnitt 11, 12 relativ groß und in dem mittleren Abschnitt 13 allmählich auf ein Minimum verringert. Eine Abmessung "d", die zwischen den Innenrändern gemessen wird, verringert sich ebenfalls in dem mittleren Abschnitt 13 allmählich auf ein Minimum. In jeder der Klebezonen 7, 8 ist eine Breite W_{C} im hinteren Abschnitt 12 größer als Breiten W_{A}, W_{B} im vorderen bzw. mittleren Abschnitt. Eine derartige Konfiguration der Klebezonen 7, 8 erlaubt es, die Einlage 1 an dem Höschen entlang dem rechten und dem linken Seitenrand um die Beine der Trägerin symmetrisch bezüglich des Schrittbereichs zu befestigen. Eine Fläche der Klebezonen 7, 8 auf der Oberfläche der Einlage 1, die zum Anlegen an das getragene Höschen bestimmt ist, kann so dimensioniert sein, daß sie im hinteren Abschnitt 12 größer ist als im vorderen und im mittleren Abschnitt 11, 13.

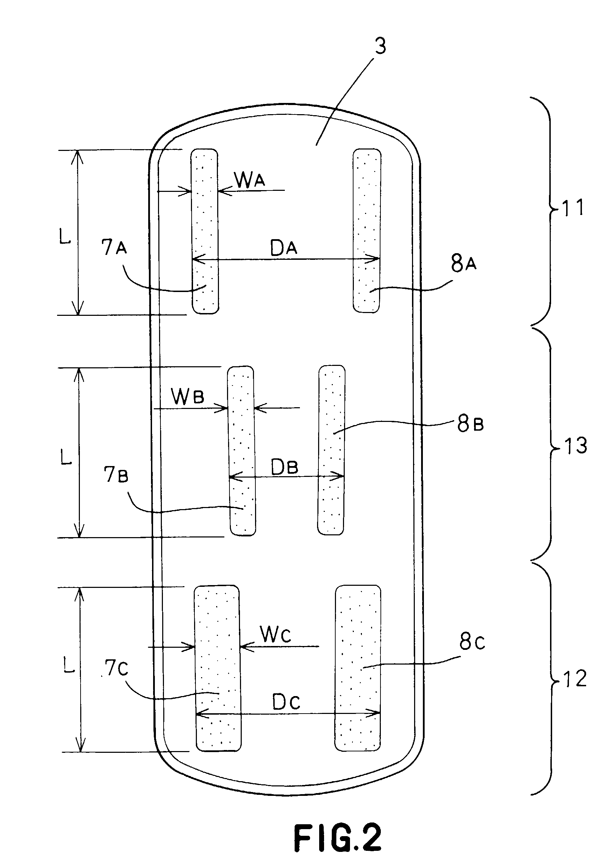

Wie Fig. 2 zeigt, sind Klebezonen entlang in Querrichtung einander gegenüberliegenden Seitenrändern der Einlage 1 jeweils in drei gerade Zonen 7_{A}, 7_{B}, 7_{C} und 8_{A}, 8_{B}, 8_{C} mit im wesentlichen derselben Länge "L" unterteilt, welche den vorderen, mittleren und hinteren Abschnitt 11, 13, 12 der Einlage 1 einnehmen. Die in Querrichtung einander gegenüberliegenden Klebezonen sind parallel zueinander im Abstand angeordnet, mit der Breite W_{A} in dem vorderen Abschnitt 11, W_{B} in dem Mittelabschnitt 13 und W_{C} in dem hinteren Abschnitt 12, wobei W_{C} größer als W_{A} bzw. W_{B} ist.

Die jeweiligen Paare von in Querrichtung einander gegenüberliegenden Klebezonen sind voneinander durch die Breite D_{A}, D_{B}, D_{C} im vorderen, mittleren bzw. hinteren Abschnitt 11, 13, 12 beabstandet, wobei D_{A} im wesentlichen gleich D_{C} ist, D_{B} jedoch kleiner ist als D_{A} und D_{C}. Demgemäß ist die Gesamtfläche der Klebezonen in dem hinteren Abschnitt 12 größer als im vorderen und im mittleren Abschnitt 11, 13, und eine zum Abtrennen derselben erforderliche Kraft entsprechend höher als im vorderen und im mittleren Abschnitt 11, 13. Alternativ ist es möglich, alle Klebezonen so zu dimensionieren, daß sie gleiche Flächen haben, und eine Klebkraft des Klebstoffs in den Zonen 7_{C}, 8_{C} so einzustellen, daß sie höher ist als in den Zonen 7_{A}, 7_{B}, 8_{A}, 8_{B}, so daß eine zum Abtrennen im hinteren Abschnitt 12 erforderliche Kraft so eingestellt werden kann, daß sie höher als in den anderen Abschnitten ist.

Die zum Abtrennen in dem hinteren Abschnitt 12 erforderliche Kraft, die durch die Anordnung sichergestellt wird, die in Form eines Beispiels durch Fig. 1 und 2 dargestellt wird, ist vorzugsweise wenigstens das 1,2-fache dieser Kraft im vorderen Abschnitt 11.

Die Einlage 1 gemäß der Erfindung kann so getragen werden, daß der Mittelabschnitt 13, der die Mindestabmessung "D" zwischen den äußeren Rändern der Klebezonen 7, 8 hat, im Schrittbereich positioniert ist, und der hintere Abschnitt 12 jeweils am Hüftbereich des Höschens positioniert ist. Die Einlage 1 ist somit in Längsrichtung richtungsgebunden und vorzugsweise mit geeigneten Einrichtungen versehen, die der Trägerin die Identifizierung dieser bestimmten Richtung der Einlage 1 erleichtern. Die Einlage 1 wird im Handel dem Verbraucher mit durch Trennpapier schützend abgedeckten Klebezonen 7, 8 geliefert. Der Klebstoff kann auf die Zonen 7, 8 so aufgetragen werden, daß flächige Verläufe, Punkte oder unterbrochen angeordnete Linien gebildet werden. Die Abmessung "d", die zwischen den Innenrändern der Klebezonen 7, 8 gemessen wird, wird vorzugsweise auf 10 Millimeter oder größer und bevorzugter auf 15 Millimeter oder größer eingestellt, um zu verhindern, daß die Klebezonen 7, 8 miteinander verklebt werden.

Eine Einlage, wie beispielsweise die Menstruationseinlage gemäß der Erfindung, kann an Höschen nicht nur um den Schrittbereich, sondern auch entlang den Seitenrändern der Beinöffnungen in der Nähe des Schrittbereichs befestigt werden, ohne der Befürchtung, daß die Einlage auch während einer aktiven Bewegung des Körpers der Trägerin verdreht werden könnte, da die zwischen den äußeren Rändern der in Querrichtung einander gegenüberliegenden Klebezonen gemessene Abmessung in dem in Längsrichtung vorderen und hinteren Abschnitt größer ist als in dem in Längsrichtung mittleren Abschnitt der Binde. Zusätzlich kann die Einlage, die durch Menstruationsflüssigkeit verschmutzt wurde, problemlos von dem Höschen in Richtung vom vorderen zum hinteren Abschnitt abgezogen werden, da die zum Trennen der Einlage erforderliche Kraft so eingestellt ist, daß sie im hinteren Abschnitt höher ist als im vorderen und im mittleren Abschnitt der Binde. Dieses Merkmal der Erfindung stabilisiert in effektiver Weise den hinteren Abschnitt der Einlage gegen eine aktive Bewegung des Körpers der Trägerin oder häufiges Hinsetzen auf und Aufstehen von einem Stuhl. Auf diese Weise ist die erfindungsgemäße Einlage komfortabel zu tragen und verschmutzt kaum den Körper oder die Kleidung der Trägerin.

## Patentansprüche

1. Saugfähige Einlage, umfassend eine flüssigkeitsdurchlässige Decklage (2), eine flüssigkeitsundurchlässige Außenlage (3), einen flüssigkeitssaugfähigen Kern (4) und eine Befestigungseinrichtung, die an der Außenlage (3) zur Befestigung der Einlage am Höschen vorgesehen ist, wobei die Befestigungseinrichtung Klebezonen (7,8) umfaßt, die entlang in Querrichtung an einander gegenüberliegenden Seiten der Einlage vorgesehen sind, wobei eine zwischen äußeren Rändern der in Querrichtung einander gegenüberliegenden Klebezonen (7,8) gemessene Abmessung (D) in einem in Längsrichtung vorderen und hinteren Abschnitt (11,12) der Einlage größer ist als in einem in Längsrichtung mittleren Abschnitt (13) der Einlage,
dadurch **gekennzeichnet**,
daß eine zum Abziehen der Einlage erforderliche Kraft in dem hinteren Abschnitt (12) höher ist als in den übrigen Abschnitten.

2. Saugfähige Einlage nach Anspruch 1,
dadurch **gekennzeichnet**,
daß eine zwischen inneren Seitenrändern der Klebezonen (7, 8) gemessene Abmessung (d) in dem mittleren Abschnitt (13) kleiner ist als in dem vorderen Abschnitt (11) und dem hinteren Abschnitt (12).

3. Saugfähige Einlage nach Anspruch 1,
dadurch **gekennzeichnet**,
daß die Klebezonen (7,8) kreisförmige Bögen beschreiben, die bezüglich einer Mittellinie konvex sind, die die Einlage in Querrichtung teilt.

4. Saugfähige Einlage nach Anspruch 1,
dadurch **gekennzeichnet**,
daß die Klebezonen jeweils in drei gerade Zonen (7_{A}, 7_{B}, 7_{C}, 8_{A}, 8_{B}, 8_{C}) mit im wesentlichen derselben Länge geteilt sind, die den vorderen, den mittleren und den hinteren Abschnitt (11, 13, 12) einnehmen und die Klebezonen (7_{A}, t_{B}, 7_{C}, 8_{A}, 8_{B}, 8_{C}), die einander in Querrichtung gegenüberliegen, parallel voneinander durch eine erste Breite in dem vorderen Abschnitt (11), eine zweite Breite in dem mittleren Abschnitt (13) und eine dritte Breite in dem hinteren Abschnitt (12) beabstandet sind, wobei die dritte Breite größer ist als die erste Breite und die zweite Breite.

## Claims

1. Absorbent pad, comprising a liquid-permeable cover layer (2), a liquid-impermeable outer layer (3), a liquid-absorbent core (4) and a fastening device, which is provided on the outer layer (3) to fasten the pad to the pants, the fastening device comprising adhesive zones (7, 8) which are provided on sides of the pad lying opposite one another in a transverse direction, and a dimension (D), measured between outer edges of the adhesive zones lying opposite one another in a transverse direction, being greater in a front and rear section (11, 12) in the longitudinal direction of the pad than in a central section (13) in the longitudinal direction of the pad,
**characterised in that**
the force necessary to pull away the pad is greater in the rear section (12) than in the remaining sections.

2. Absorbent pad according to claim 1,
**characterised in that**
a measured dimension (d) between inner side edges of the adhesive zones (7,8) is smaller in the central section than in the front section (11) and the rear section (12).

3. Absorbent pad according to claim 1,
**characterised in that**
the adhesive zones (7,8) describe circular arcs, which are convex in respect of a central line, which divides the pad in the transverse direction.

4. Absorbent pad according to claim 1,
**characterised in that**
the adhesive zones are respectively divided in three straight zones (7_{A}, t_{B}, 7_{C}, 8_{A}, 8_{B}, 8_{C}) of essentially the same length which take up the front, the central and the rear sections (11, 13, 12), and in that the adhesive zones (7_{A}, t_{B}, 7_{C}, 8_{A}, 8_{B}, 8_{C}), which lie opposite one another in the transverse direction, are parallel and spaced from one another by a first width in the front section (11), a second width in the central section (13) and a third width in the rear section (12), the third width being greater than the first width and the second width.

## Revendications

1. Garniture cataméniale absorbante, comprenant une couche de revêtement (2) perméable aux liquides, une couche extérieure (3) imperméable aux liquides, un corps central (4) absorbant les liquides, et un système de fixation prévu sur la couche extérieure (3) pour permettre la fixation de la garniture à une petite-culotte, le système de fixation comprenant des zones adhésives (7, 8) longeant des faces transversalement opposées de la garniture, une dimension (D) mesurée entre des bords extérieurs des zones adhésives (7, 8) transversalement opposées étant plus grande dans une portion (11, 12) longitudinalement antérieure et postérieure de la garniture que dans une portion longitudinalement médiane (13) de la garniture, caractérisée en ce que la force nécessaire à l'arrachement de la garniture est plus grande dans la portion postérieure (12) que dans les autres portions.

2. Garniture cataméniale absorbante selon la revendication 1, caractérisée en ce qu'une dimension (d) mesurée entre des bords latéraux intérieurs des zones adhésives (7, 8) est plus petite dans la portion médiane (13) que dans la portion antérieure (11) et dans la portion postérieure (12).

3. Garniture cataméniale absorbante selon la revendication 1, caractérisée en ce que les zones adhésives (7, 8) décrivent des arcs de cercle qui sont de configuration convexe par rapport à un axe qui partage la garniture dans le sens transversal.

4. Garniture cataméniale absorbante selon la revendication 1, caractérisée en ce que les zones adhésives sont partagées chacune en trois zones droites (7_{A}, 7_{B}, 7_{C}, 8_{A}, 8_{B}, 8c) sensiblement de longueur identique, qui occupent les portions antérieure, médiane et postérieure, et en ce que les zones adhésives (7_{A}, 7_{B}, 7_{C}, 8_{A}, 8_{B}, 8_{C})qui s'opposent mutuellement dans le sens transversal, sont espacées parallèlement les unes des autres par une première largeur dans la portion antérieure (11), une deuxième largeur dans la portion médiane (13), et une troisième largeur dans la portion postérieure (12), la troisième largeur étant plus grande que les première et deuxième largeurs.
